# EUROPEAN PATENT SPECIFICATION

(11) **EP 0 523 085 B1**
(45) Date of publication and mention of the grant of the patent: **21.08.1996**
(21) Application number: 91906565.6
(22) Date of filing: 14.03.1991
(51) Int. Cl.: C07C 325/00, C07C 261/00, C07C 233/00, C07D 265/30, C07C 233/36

(54) **PEROXY ACID BLEACH PRECURSORS AND DETERGENT COMPOSITIONS CONTAINING THEM**
VORLÄUFER FÜR DIE BLEICHE MIT PEROXYSÄURE UND REINIGUNGSMITTELZUSAMMENSETZUNGEN DIE DIESE VORLÄUFER ENTHALTEN
PRECURSEURS DE BLANCHISSAGE A L'ACIDE PEROXY ET COMPOSITIONS DETERGENTES CONTENANT CES PRECURSEURS

(30) Priority: 31.03.1990 GB 9007293
(43) Date of publication of application: 20.01.1993
(73) Proprietor: THE PROCTER & GAMBLE COMPANY, Cincinnati,Ohio 45202 (US)
(72) Inventor: HARDY, Frederick Edward, Ponteland,Newcastle upon Tyne NE20 9ES (GB)
(74) Representative: Canonici, Jean-Jacques
(86) International application number: US9101700
(87) International publication number: WO9115474

(56) References cited:
- US-A- 1 737 458
- US-A- 4 481 150
- CHEMICAL ABSTRACTS, vol. 105, no. 12, 22 September 1986, Columbus, Ohio, US; abstract no. 102417v, H. VERPLAETSE ET. AL. 'Screening of chelating agents for chemolysis' page 320 ;
- CHEMICAL ABSTRACTS, vol. 100, no. 13, 26 March 1984, Columbus, Ohio, US; abstract no. 102940, 'Sulfonyl chlorides' page 611 ;

## Description

This invention relates to new compositions of matter comprising organic compounds adapted to form aliphatic peroxycarboxylic acids when exposed to alkaline aqueous solutions of hydrogen peroxide. More especially this invention concerns such compounds having enhanced solubility at water temperatures approximating those of the cold water supply i.e. 10-15°C.

Organic compounds adapted to form peroxycarboxylic acid bleaches are more commonly referred to as bleach activators, and are incorporated into laundry detergent compositions containing inorganic perhydrate salts to provide bleaching of oxidisable stains on fabrics at wash-liquor temperatures of 60°C and below. Such compounds comprise one or more acyl groups, each of which forms a potential peroxyacid precursor bonded to a leaving group, the parent acid of which has a pKa in the range from 6 to 14. Leaving groups whose parent acids have low pKa values allow perhydrolysis of the activator under mild conditions to form the peroxy acid species, but such bleach activator compounds tend to be of low stability in detergent compositions. Bleach activators with leaving groups whose parent acids have high pKa values are more stable but lose selectivity towards perhydrolysis, saponifying extensively and giving low yields of peroxyacid.

A large number of bleach activator compounds have been disclosed by the art but only a relatively small number have been exploited commercially. Examples of bleach activators that have been employed commercially include sulphonated phenyl esters as disclosed in BP 864,798, 963,135 and EP-A-0120591, acetylated glycourils as disclosed in BP 1,246,338 and N,N-diacylated nitrogen-containing compounds as disclosed in BP 855,735 and 907,356.

US-A-1,737,458 describes quaternary ammonium compounds of acylated alkylenediamines having soaping properties. US-A-4,481,150 describes surface active hydroxyalkylaminosulfonic acids.

The need to maintain an acceptable level of bleach activator over the expected shelf life of the detergent composition has traditionally resulted in stability being favoured at the expense of solubility and reactivity. The bleach activators in widespread commercial use have thus been crystalline, relatively hydrophobic materials with a correspondingly low aqueous solubility, typified by the most widely used material, tetra acetyl ethylene diamine (TAED) which has a low (approximately 0.2%) solubility in water at room temperature (20°C). This low solubility has hitherto been tolerable in a laundry washing practice where the rates of solution of product ingredients in general, and bleach components in particular, have not been of critical importance.

Laundry washing practice in European countries has traditionally involved the use of wash temperatures near to, or at, the boil whereas in Japan and a number of Latin American countries there has always been a significant level of fabric washing at so-called cool temperatures (=20°C). The detergent product formulations for these markets have therefore differed from those in Europe because of this. These differences have tended to restrict the growth in the usage of bleach activators outside of Europe.

However, the increase in detergent bleach activator usage in Europe has been accompanied by a progressive decrease in the temperatures at which fabrics are laundered, associated with the increase in coloured fabric usage. There is also an increasing interest by consumers in saving energy in the washing process, reinforced by a trend towards reduced wash-water volumes. Moreover, both in Japan and Europe there has recently been a growth in the use of concentrated granular detergent products having higher density and less volume than traditional granular detergents. All of these changes have tended to increase the stress on the solution characteristics of the product ingredients.

A further recent innovation has been the delivery of solid detergent products to the wash-liquor by dispensing devices placed within the drum of the washing machine. This latest development, whilst largely eliminating loss through the conventional dispensing system, particularly in the machine sump, places a premium on the rapid and complete solution of the materials making up the bleach component. High local concentrations of bleaching species arising from slow or incomplete solution of the components can give rise to a range of product fabric discolouration problems.

A need thus exists to improve the solubility of bleach activators generally, without an unacceptable sacrifice in their stability in detergent products.

This need has been recognised in the art and EP-A-0333248 discloses peroxyacid bleach precursors of the sulphonated phenyl ester type that are asserted to be suitable for domestic laundry detergent compositions and to have improved overall bleaching performance at the wash temperature region of from ambient to about 40°C. The precursors of EP-A-0333248 are provided with an additional substituent group on the benzene nucleus in the 1-position relative to the 0-acyl moiety, this substituent group being a methyl, ethyl ethoxy or ethoxy group.

Nevertheless it would still be desirable to improve the low temperature solution characteristics of the most widely used class of bleach activator, viz. the acylated diamines.

Only one of the acyl groups in a tetra acylated alkylene diamine cleaves from the molecule to provide a peroxyacid precursor grouping and the tetraacylated molecule can be regarded as two separated N-diacyl moieties, each of which is a potential source of the precursor group. Tetraacylated alkylene diamines are conventionally made in a multi-stage process in which the diacylated alkylene diamine is an intermediate but it has not hitherto been found feasible to introduce a water-solubilising group into the diamine structure. One of the principal reasons for this has been that the incorporation of a group conferring aqueous solubility interferes with the process in which the N-diacyl grouping is formed.

However the Applicants have now surprisingly found that it is possible to synthesise a molecule incorporating both a N-diacyl group functionality, (and hence peroxyacid precursor capability) and a moiety promoting improved solution characteristics in cool (i.e. 10°C-40°C) water.

According to one aspect of the present invention there is provided a water-soluble peroxyacid bleach precursor having the general formula: 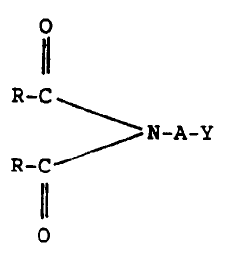 wherein each
R is CH₃- or C₂H₅-
A is C₂-C₆ alkylene or a phenylene group
Y is
   - OSO₃M+
   - SO₃ M+
   - N+R₁R₂R₃X- 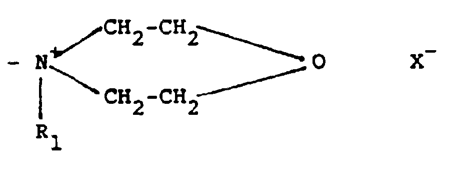 wherein M⁺ is hydrogen alkali metal or alkaline earth metal, R₁ is C₁-C₂₀ alkyl, R₂ and R₃ are independently C₁-C₄ alkyl and X- is halide or methosulphate.

According to another aspect of the invention there is provided a method of making a peroxyacid bleach precursor of the type defined hereinbefore. Further aspects of the invention include bleach and detergent compositions incorporating the hereinbefore defined peroxyacid bleach precursors and a method of bleaching oxidisable stains on fabrics by means of such compositions

For the purposes of the present invention the solubility of a precursor in water is defined as the weight of the compound that can be dissolved in 100ml distilled water at 10°C, expressed on a percentage basis. Precursors in accordance with the invention have water solubilities of at least 25% by weight, more preferably at least 50% and most preferably at least 75%.

Compositions incorporating inorganic perhydrate bleaches and peroxyacid bleach precursors in accordance with the invention show enhanced bleaching capability relative to similar compositions containing prior art acylated alkylene diamines at ambient temperatures, viz. 10-25°C. Furthermore detergent compositions containing the highly soluble precursors of the invention show a reduced tendency to cause localised colour and fabric damage when delivered from a dispensing device located in the drum of a domestic washing machine, particularly at wash temperatures of 60°C and below.

In its broadest aspect the invention comprises a peroxyacid bleach precursor having the general formula: 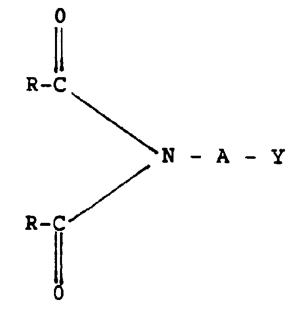 wherein each
R is CH₃- or C₂H₅-
A is C₂-C₆ alkylene or a phenylene group
Y is
   - OSO₃M+
   - SO₃M+
   - N+R₁R₂R₃X- 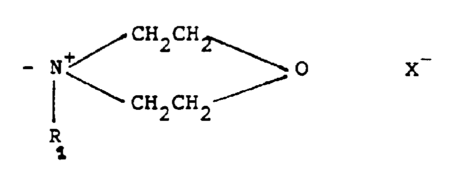 wherein M⁺ is hydrogen, alkali metal or alkaline earth metal, R₁ is a C₁-C₂₀ alkyl group, R₂ & R₃ are independently C₁-C₄ alkyl groups and X- is halide or methosulphate.

Preferably the R, R_{1'} R₂ & R₃ groups are methyl groups and A is a C₂-C₃ alkylene group.

Where Y is anionic, the preferred anionic radical is a sulphonate group, A is an ethylene group and M⁺ is sodium or more preferably potassium. The most preferred material of this type is the potassium salt of di-N-acetyl taurine.

This class of materials can be prepared by heating an amino alkyl sulphonate with triethylamine in an excess of acetic or propionic anhydride. The desired product is then precipitated from the mixture by adding an alkali or alkaline earth metal acetate dissolved in acetic acid.

Where Y is cationic, the preferred group is a trimethyl ammonio halide, preferably bromide or chloride and A is a n-propylene moiety. The preferred material of this type is N,N-diacetyl trimethyl ammonio propylamine bromide or chloride.

The cationic materials can be prepared by di-N-acylation of a primary-tertiary diamine with acetic or propionic anhydride followed by quaternisation with an alkyl halide.

Alternatively the primary-tertiary diamine can be heated with a lower alkyl acetate or propionate to introduce the first acyl group followed by treatment of the reaction product with the corresponding acyl chloride to form the di lower alkylamino diacyl amide. This material is then quaternised with an alkyl halide to give the final product.

The preparation of exemplary compounds according to the invention will now be described.

### The preparation of potassium N,N-diacetylaminoethane sulphonate.

The following materials were charged, in the order shown, to a 500ml conical flask fitted with a magnetic stirrer, condenser, dropping funnel, thermometer and CaCl₂/sodalime tubes:
(i) 40.43g triethylamine (0.40moles) supplied by British Drug Houses
(ii) 50g taurine (0.40 moles) supplied by Sigma Chemical Co.
(iii) 200ml. (216g, 2.1 moles) AnalaR acetic anhydride (supplied by British Drug Houses Ltd.)

Agitation was maintained throughout and the acetic anhydride was added over an 8 minute period. After 20 minutes the temperature had risen gradually to 33°C and the taurine had begun to dissolve. Heating, via an oil-bath, was then applied to raise the temperature to reflux (141°C) over a period of 1 hour during which time the solid dissolved completely to give a dark brown solution.

After refluxing for one hour a sample was removed, evaporated, and treated by washing and stripping with cyclohexane. This sample was found to have a significant -NH-lnfraRed peak indicating that the reaction was incomplete. The main batch was allowed to reflux for a further hour. A sample removed and treated as above was found to have a less significant -NH-lnfraRed peak. The batch was then removed from the oil-bath and allowed to cool.

The product was then reheated to 130°C and a total of 39.34g potassium acetate in 41.65g boiling acetic acid was added with agitation. The mix was allowed to cool with stirring when a creamy brown solid precipitated.

This was filtered under vacuum and the precipitate was slurried in ether, refiltered, and washed with ether before being dried in a dessicator over P₂0, and paraffin wax. A total dried weight of 61.58g was obtained of a material found by NMR and InfraRed spectra to comprise 82% of the N,N-diacetyl- and 18% of the N-monoacetyl-derivative of potassium amino ethane sulphonate. This represented a yield of 62.1 % of that obtainable theoretically

The water solubility of this material was determined by cooling 2.3g distilled water to 10°C and adding the precursor until no further material would dissolve. A slight excess was then added and the temperature raised to 25°C to give complete solution before recooling to 10°C, ensuring that crystallisation of the precursor took place from a saturated solution. The amount of the precursor dissolved under these conditions was found to be 4.71 g i.e. a solubility of 66.6% at 10°C.

### The preparation of N,N-diacetyl trimethylammonio propylamine bromide.

### A) "One Pot" two stage synthesis

20.44 grams (0.2 moles) of 3-dimethylaminopropylamine supplied by Hoechst were weighed into a 150ml round-bottomed flask surrounded by an ice bath and fitted with a magnetic stirer, a dropping funnel and a short Vigreaux fractionation column. 61.25 grams (0.6 mole) of acetic anhydride (supplied by British Drug Houses Ltd.) were carefully added to the flask via the dropping funnel whilst stirring vigorously as this addition was highly exothermic. When all the acetic anhydride had been added, the ice-bath was replaced with a thermomantle and the acetic acid produced in the reaction removed by distillation at atmospheric pressure. The distillate was monitored by means of a Gas chromatograph and any acetic anhydride lost to the distilling flask was replenished via the dropping funnel. Distillation was continued until no more acetic acid could be detected in the distillate. After cooling to room-temperature, the fractionation column was removed and replaced with a sub-ambient temperature (acetone/C0₂) reflux condenser fitted with a calcium chloride drying-tube. With vigorous stirring at room-temperature 20.9 gms (0.22 mole) of methyl bromide (supplied by British Drug Houses Ltd.) pre-cooled to -5°C, were added to the flask via the dropping funnel. Solid product was formed in a few minutes and the resulting suspension was stirred at room-temperature for ½ hour. The reaction mixture was filtered using vacuum filtration and the collected solid was washed with ice cold acetone and dried to constant weight in a vacuum desiccator over phosphorus pentoxide. This yielded 42 grams of product which by a perhydrolyis test had a purity of greater than 95%.

### B) Three stage synthesis

### Step 1

192 gms (1.88 moles) of 3-dimethylaminopropylamine (supplied by Aldrich) were weighed into a 1 litre round-bottomed flask fitted with a magnetic stirrer and a reflux condenser carrying a CaC'₂/Carbosorb tube. Next, 380gms (4.32 moles) of AnalaR ethyl acetate (supplied by British Drug Houses) were added and the apparatus was set to reflux with stirring. This was continued for seven days after which the pale yellow liquid reaction product was subjected to fractional distillation. The first fraction, removed under atmospheric pressure at a pot temperature of 77°C, was 215g of ethyl acetate which was discarded. The second fraction, removed under atmospheric pressure at temperatures of from 78°C to 130°C, was a mixture of ethyl acetate and 3-dimethylamino propylamine, 128.2gms which was also discarded The system was then allowed to cool before distillation was continued at reduced pressure (= 10mm). At 57°C, a further 32.46 gms comprising more starting amine material was removed. Two further fractions, collected at pot temperatures from 80°C to 130°C and 130°C, weighed 13.5g and 167.7g respectively and comprised substantially pure 3-dimethylamino propyl acetamide of purity 96.5%.

### Step 2

54.4g of acetyl chloride were weighed into a 250ml round-bottomed flask fitted with a reflux condenser, topped by a CaCI₂ tube, and a magnetic stirrer. The drying-tube was temporarily removed and 50g of the product of Step 1 was added to the flask in stages via the condenser with stirring of the flask contents. A controlled exothermic reaction took place but no cooling was required. When all the monoacetamide had been added the reaction mixture became a thick, dark coloured, suspension. The contents of the flask were then refluxed for three hours using an oil-bath, after which the condenser was removed and the volatiles were allowed to boil off, maintaining stirring of the flask contents. During this operation, all of the solid material liquified and the reaction mixture became a mobile black liquid. This was cooled to room temperature, taken up in chloroform and transferred to a separating funnel.

The chloroform solution was washed successively with saturated solutions of sodium carbonate and sodium chloride, discarding the aqueous liquors, and was then dried over anhydrous sodium sulphate. Evaporation of the dried solution yielded 55.3g of a black liquid which was distilled under reduced pressure (1-2mm) using an Edwards Vacuum Pump and a vigreaux column. The clear colourless fraction distilling at 74-75°C weighing 48.92g was collected and the residue discarded. GLC analysis showed the product to be 3-dimethylaminopropyl diacetamide with a purity of 92.3%.

### Step 3

10g of the product of Step 2 was weighed into a small conical flask fitted with a magnetic stirrer and CaCI₂ drying-tube and surrounded by an ice-bath. 100m1s acetone was added and the resulting solution was stirred at 0°C whilst 7.7g (0.5M) of AnalaR methyl bromide in 25mls acetone was added by the temporary removal of the drying-tube. After a few minutes a white suspension was formed and after a further 15 minutes the flask was removed from the ice-bath and allowed to warm to room temperature over a half hour period. The suspension was then filtered and the filter cake washed with ice-cold acetone. The resulting white solid was dried over P205 in a vacuum desiccator to constant weight. The yield was 13.3g of N,N-diacetyl trimethyl ammoniopropylamine bromide and a perhydrolysis test showed this material to have 97.3% activity.

A determination of the solubility of this product in distilled water at 10°C using the technique previously described gave a solubility of 75.6%.

The present invention also relates to bleach additive compositions incorporating the peroxyacid precursors defined hereinbefore, that are adapted to provide bleaching of oxidisable stains on surfaces, particularly fabric surfaces, when mixed with an alkaline aqueous source of hydrogen peroxide at temperatures of 60°C and below.

Such compositions can be in liquid, pasty or solid form. Aqueous compositions can be formed with precursor concentrations of up to 50% by weight depending on the type and level of other components of the composition but preferably contain from 1 % to 25%, more preferably from 3% to 15%, by weight of precursor. Optional additional components can include surfactants, chelating agents, bleach-stabilising agents and other compatible detergent composition additives.

Pasty or solid bleach additive compositions can incorporate higher levels of the peroxyacid precursors of the present invention than the compositions in liquid form. Typical pasty or solid compositions may comprise from 50% to 95% precursor. Whilst pasty compositions will tend to be similar to liquid compositions in constitution, solid compositions may include a wider variety of other components and may take various physical forms such as granules, noodles, tablets or flakes.

Solid compositions may comprise the precursor in combination with a compatible substrate and/or may include an agglomerating or binding agent which may be water, a water-soluble organic polymeric material, a hydratable salt, an organic surfactant or a mixture of any of these. A preferred particu late form of such a combination is an agglomerate or extrudate.

Hydratable salts are principally inorganic in character and suitable materials include clays and other natural and synthetic aluminosilicates, as well as hydratable salts such as phosphates, carbonates and sulphates. Suitable water-soluble organic materials include polyethylene glycols of MWT 4,000-10,000, carboxymethyl cellulose derivatives, and acrylate and methacrylate homo- and co-polymers of MWT 4,000-40.000. Surfactants useful as binding and/or agglomerating agents include ethoxylated C₁₂-C₁₈ alcohols, particularly tallow chain-length alcohols condensed with from 25 to 80 moles of ethylene oxide per mole of alcohol, alkyl phenols and fatty acids, alkyl polyglucosides and alpha-sulphonated esters of Cᵢ₂-Cᵢ₈ fatty acids.

Preferred methods of making a solid composition particulate from a mixture of precursor and an organic material or a hydratable inorganic salt are disclosed in the Applicants' Published European Application Nos 0062523 and 0106634.

Additive products in accordance with this aspect of the present invention may also comprise the precursor alone in combination with a carrier such as a container or a flexible non-particulate substrate.

Additive products enclosed in bags or containers are manufactured such that the containers prevent egress of their contents when dry, but are adapted to release their contents on immersion in an aqueous solution.

Usually the container will be flexible, such as a bag or pouch. The bag may be of fibrous construction coated with a water-impermeable protective material so as to retain the contents, such as is disclosed in European published Patent Application No. 0018678. Alternatively it may be formed of a water-insoluble synthetic polymeric material provided with an edge seal or closure designed to rupture in aqueous media as disclosed in European published Patent Application Nos. 0011500, 0011501, 0011502, and 0011968. A convenient form of water-frangible closure comprises a water-soluble adhesive disposed along and sealing one edge of a pouch formed of a water-impermeable polymeric film such as polyethylene or polypropylene.

An alternative form of the additive product comprises a compound as hereinbefore defined in water-releasable combination with a non-particulate flexible substrate in a weight ratio of 1:10 to 30: 1. Additive products of this type are disclosed in British Patent Specification Nos. 1,586,769 and 2040983B and also in British Published Patent Application No.2125453.

A highly preferred execution of this type of additive product utilises a flexible sheet so as to make it compatible with the movement of the fabrics in the washing machine and to facilitate its handling during manufacture of the product. Preferably the sheet is water-pervious i.e. water can pass from one surface of the sheet to the opposite surface and, for film-type substrates, perforation of the sheet is desirable. The most preferred form of the substrate is a sheet of woven or non-woven fabric or a thin sheet of cellular plastic material. Woven fabric sheets can take the form of a plain- weave natural or synthetic fibre of low fibre count/unit length, such as is used for surgical dressings, or of the type known as cheese-cloth.

Loading limitations on sheet-type substrates limit the amount of precursor compound that can be applied to the sheet and, in practice, the weight ratio of precursor compound:sheet substrate normally lies within the range from 1:2 to 10:1.

Variations on the above sheet substrate product forms are also contemplated by the present invention. For example, laminated sheet products can be employed in which a central layer is impregnated and/or coated with a composition incorporating the bleach precursor and then one or more outer layers are applied to produce a fabric-like aesthetic effect. The layers may be sealed together so as to remain attached during use or may separate on contact with water to facilitate the release of the coated or impregnated material.

An alternative laminate form comprises one layer embossed or deformed to provide a series of pouch-like containers into each of which the bleach precursor and optionally other detergent components are deposited in measured amounts, with a second layer overlying the first layer and sealed thereto in those areas between the pouch-like containers where the two layers are in contact. The bleach precursor and any accompanying components may be deposited in particulate, paste or molten form and the laminate layers should prevent egress of the contents of the pouch-like containers prior to their addition to water. The layers may separate or may remain attached together on contact with water, the only requirement being that the structure should permit rapid release of the contents of the pouch-like containers into solution. The number of pouch-like containers per unit area of substrate is a matter of choice but will normally vary between 500 and 25,000 per square metre.

Suitable materials which can be used as a flexible substrate in this aspect of the invention include, among others, sponges, paper, and woven and non-woven fabrics.

In addition to the precursor compounds of the present invention, products and compositions made in accordance with the invention may optionally contain any of the organic peroxy acid bleach precursors known in the art. A detailed disclosure of such precursors is provided in British Patent Specification Nos. 2040983 and 2143231. Blends of the precursors of the present invention with branched C_{S}-C_{lO} acyl oxybenzene sulphonate or carboxylate precursors are preferred, although mixtures can be formed with other peracetic acid precursors including tetra acetyl ethylene diamine, tetra acetyl methylene diamine, tetra acetyl glycouril, sodium p-acetoxybenzene sulphonate, penta acetyl glucose and octa acetyl lactose. Moreover, the invention also contemplates blends with e.g. peroxybenzoic and peroxyphthalic acid precursors where different combinations of bleaching properties are required.

Any solid mixtures of precursors will normally be processed in a common particulate although the invention also contemplates their addition as separate particulate entities.

The level of usage of the bleach additive composition will naturally be dependent on a number of factors, e.g. the size of the fabric load in the machine, the level of bleaching performance desired, the amount of perhydroxyl ion in the wash solution, the bleaching efficacy of the organic peroxy species derived from the precursor and the efficiency of conversion of the precursor into that peroxy species. It is conventional with inorganic peroxy bleaches to provide a level of available oxygen in solution from 50 ppm to 350 ppm by weight for heavy-duty laundry purposes. When using organic peroxy bleaches, the level of available oxygen provided by the organic peroxy compound normally lies in the range from 1 ppm to 50 ppm. Levels of from 1.5 ppm to 10 ppm are considered appropriate under conventional US washing conditions while under European washing conditions, levels of from 15 ppm to 30 ppm are more commonly used

For a machine utilising wash-liquor volumes of from 15 to 30 litres, such a level of available oxygen requires the delivery of from 2 gr to 20 gr of organic peroxy compound percursor, assuming quantitative conversion.

Bleaching compositions according to the present invention can also comprise the combination of particulates incorporating the hereinbefore defined precursors with the spray-dried portion of a detergent composition and the other dry-mix ingredients such as enzymes, inorganic peroxygen bleaches and suds-suppressors. The spray-dried portion of the composition normally provides the alkalinity and the inorganic peroxygen bleach provides the source of hydrogen peroxide for the production of the organic peroxycarboxylic acid from the precursor.

It will be appreciated however that the detergent composition to which the precursor particulates are added may itself be made in a variety of ways such as dry-mixing, agglomeration, extrusion, flaking etc, such ways being well known to those skilled in the art and not forming part of the present invention.

The source of hydrogen peroxide can be provided by any of the commercially available inorganic peroxygen bleaches and also by certain hydrogen peroxide adducts.

Suitable inorganic peroxygen bleaches include sodium perborate mono and tetra hydrate, sodium percarbonate, sodium persilicate and the clathrate 4Na₂S0₄ : 2H₂0₂ : 1 NaCi. A separate source of alkalinity is required for clathrate materials and for stability reasons this should preferably be kept physically separated from the hydrogen peroxide source by e.g. enrobing or encapsulating the latter. The hydrogen peroxide source will normally be present in an amount of from 1% to 40%, more preferably from 5% to 35% by weight of the composition and will most frequently be present in an amount of from 10% to 30% by weight.

Preferred detergent compositions, in accordance with this aspect of the invention, will include those components commonly included in heavy-duty laundry detergents such as surfactants, suds-suppressing agents, detergent builders, chelating agents, soil-suspending and anti-redeposition agents, optical-brightening agents, fabric-softening agents, enzymes, photoactivated bleaches, colours and perfumes.

A wide range of surfactants can be used in the detergent compositions. A typical listing of anionic, nonionic, ampholytic and zwitterionic classes and species of these surfactants is given in U.S. P. 3,664,961 issued to Norris on May 23, 1972.

Mixtures of anionic surfactants are particularly suitable herein, especially mixtures of sulphonate and sulphate surfactants in a weight ratio of from 5:1 to 1:2, preferably from 3:1 to 2:3, more preferably from 3:1 to 1:1. Preferred sulphonates include alkyl benzene sulphonates having from 9 to 15, especially 11 to 13 carbon atoms in the alkyl radical, and alpha-sulphonated methyl fatty acid esters in which the fatty acid is derived from a C₁₂-C₁₈ fatty source preferably from a C₁₆-C₁₈ fatty source In each instance the cation is an alkali metal, preferably sodium. Preferred sulphate surfactants are alkyl sulphates having from 12 to 18, preferably 16 to 18 carbon atoms in the alkyl radical, optionally in admixture with ethoxy sulphates having from 10 to 20, preferably 10 to 16 carbon atoms in the alkyl radical and an average degree of ethoxylation of 1 to 6. The cation in each instance is again an alkali metal cation, preferably sodium.

One class of nonionic surfactants useful in the present invention are condensates of ethylene oxide with a hydrophobic moiety to provide a surfactant having an average hydrophilic-lipophilic balance (HLB) in the range from 8 to 17, preferably from 9.5 to 13.5, more preferably from 10 to 12.5. The hydrophobic (lipophilic) moiety may be aliphatic or aromatic in nature and the length of the polyoxyethylene group which is condensed with any particular hydrophobic group can be readily adjusted to yield a water-soluble compound having the desired degree of balance between hydrophilic and hydrophobic elements.

Especially preferred nonionic surfactants of this type are the C₉-Cᵢ₅ primary alcohol ethoxylates containing 3-8 moles of ethylene oxide per mole of alcohol, particularly the C₁₄-C₁₅ primary alcohols containing 6-8 moles of ethylene oxide per mole of alcohol and the C12-C14 primary alcohols containing 3-5 moles of ethylene oxide per mole of alcohol.

Another class of nonionic surfactants comprises alkyl polyglucoside compounds of general formula 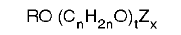 wherein Z is a moiety derived from glucose; R is a saturated hydrophobic alkyl group that contains from 12 to 18 carbon atoms; t is from 5 to 10 and n is 2 or 3; x is from 1.3 to 4, the compounds including less than 10% unreacted fatty alcohol and less than 50% short-chain alkyl polyglucosides. Compounds of this type and their use in detergent compositions are disclosed in EP-B 0070074, 0070077, 007599-6 and 0094118.

A further class of surfactants are the semi-polar surfactants such as amine oxides. Suitable amine oxides are selected from mono C₈-C₂₀, preferably C₁₀-C₁₄ N-alkyl or alkenyl amine oxides and propylene-1,3-diamine dioxides wherein the remaining N-positions are substituted by methyl, hydroxyethyl or hydroxypropyl groups.

Cationic surfactants can also be used in the detergent compositions herein and suitable quaternary ammonium surfactants are selected from mono C₈-C₁₆, preferably C₁₀-C₁₄ N-alkyl or alkenyl ammonium surfactants wherein remaining N-positions are substituted by methyl, hydroxyethyl or hydroxypropyl groups.

Mixtures of surfactant types are preferred, more especially anionic-nonionic and also anionic-nonionic-cationic mixtures. Particularly preferred mixtures are described in British Patent No. 2040987 and European Published Application No. 0087914. The detergent compositions can comprise from 1 %-70% by weight of surfactant, but usually the surfactant is present in an amount of from 1% to 20%, more preferably from 5-15% by weight.

Representative suds-suppressing agents are materials of the silicone, wax, vegetable and hydrocarbon oil and phosphate ester varieties. Suitable silicone suds-controlling agents include polydimethylsiloxanes having a molecular weight in the range from 200 to 200,000 and a kinematic viscosity in the range from 20 to 2,000,000 mm²/s (cSt), preferably from 3000 to 30,000 mm²/s (cSt), and mixtures of siloxanes and hydrophobic silanated (preferably trimeth- ylsilanated) silica having a particle size in the range from 10 millimicrons to 20 millimicrons and a specific surface area above 50 ₘ2_{/}g.

Suitable waxes include microcrystalline waxes having a melting point in the range from 65°C to 100°C, a molecular weight in the range from 4,000-10,000, and a penetration value of at least 6, measured at 77°C by ASTM-D1321, and also paraffin waxes, synthetic waxes and natural waxes. Suitable phosphate esters include mono- and/or di-Cᵢ₆-C₂₂ alkyl or alkenyl phosphate esters, and the corresponding mono- and/or di alkyl or alkenyl ether phosphates containing up to 6 ethoxy groups per molecule.

Suds-suppressors are normally included at levels of from 0.01% to 5% by weight of the composition, dependent on the type of suds-suppressor used, more commonly 0.1% to 2% by weight.

A highly preferred component of detergent compositions in accordance with the invention is one or more detergent builder salts which may comprise up to 90% of the composition, more typically from 10% to 70% by weight thereof. Suitable detergent builder salts useful herein can be of the polyvalent inorganic and polyvalent organic types, or mixtures thereof. Non-limiting examples of suitable water-soluble, inorganic alkaline detergent builder salts include the alkali metal carbonates, borates, phosphates, pyrophosphates, tripolyphosphates and bicarbonates.

Examples of suitable organic alkaline detergency builder salts are water-soluble polycarboxylates such as the salts of nitrilotriacetic acid, lactic acid, glycollic acid and ether derivatives thereof as disclosed in BE -A- 821,368, 821,369 and 821,370; succinic acid, malonic acid, (ethylenedioxy)diacetic acid, maleic acid, diglycollic acid, tartaric acid, tartronic acid and fumaric acid; citric acid, aconitic acid, citraconic acid, carboxymethyloxysuccinic acid, lactox- ysuccinic acid and 2-oxy-1,1,3-propane tricarboxylic acid oxydisuccinic acid, 1,1,2,2-ethane tetracarboxylic acid, 1,1,3,3-propane tetracarboxylic acid and 1,1,2,3-propane tetracarboxylic acid; cyclopentane cis, cis,cis-tetracarboxylic acid, cyclopentadiene pentacarboxylic acid, 2,3,4,5-tetrahydrofuran-cis, cis, cis-tetracarboxylic acid, 2,5-tetrahydrofuran-cis-dicarboxylic acid, 1,2,3,4,5,6-hexane-hexacarboxyiic acid, mellitic acid, pyromellitic acid and the phthalic acid derivatives disclosed in GB -A- 1,425,343.

A further class of builder salts is the insoluble alumino silicate type which functions by cation exchange to remove polyvalent mineral hardness and heavy-metal ions from solution. A preferred builder of this type has the formulation Na_{z}(A1O₂)_{z}(SiO₂)y.xH₂O wherein z and y are integers of at least 6, the molar ratio of z to y is in the range from 1.0 to 0.5 and x is an integer from 15 to 264. Compositions incorporating builder salts of this type form the subject of GB-A-1,429,143 published March 24, 1976, DE-A-2,433,485 published February 6, 1975 and DE-A-2,525,778 published January 2, 1976.

Mixtures of organic and/or inorganic builder salts can be used herein. A preferred mixture comprises Aluminosilicate carbonate and citrate salts in a total amount of from 20 to 60% by weight of the composition with the weight ratio of Aluminosilicate to citrate being in the range from 2:1 to 1:2.

An alkali metal, or alkaline earth metal, silicate can also be present. The level of alkali metal silicate is preferably from 3% to 15% by weight. Suitable silicate solids have a molar ratio of Si0₂/alkali metal₂0 in the range from 1.0 to 3.3, more preferably from 1.5 to 2.4. For detergent compositions in which the, or the major detergent builder salt is an insoluble aluminosilicate such as zeolite A, X or P, it is preferred that the zeolite and silicate be kept separate e.g. in different particulates. This can be achieved in several ways, e.g. by producing separate spray-dried particulates, only one of which contains the silicate, or more preferably by incorporating the zeolite in the spray-dried portion of the composition and adding the silicate as a dry mixed solid ingredient. Silicates with an Si0₂/alkali metal ₂0 ratio in the range 2.0 - 2.2 are most suitable for this purpose.

A wide variety of chelating agents can be incorporated including the alkylene amino carboxylate and phosphonate acids and their salts as disclosed in US Patent Nos. 2,599,807, 3,213,030, 3,433,021 and 3,292,121. Preferred chelating agents include nitrilotriacetic acid (NTA), nitrilotrimethylene phosphonic acid (NTMP), ethylene diamine tetra methylene phosphonic acid (EDTMP), diethylene triamine penta methylene phosphonic acid (DETPMP), and diethylene triamine penta acetic acid (DTPA) and these are incorporated in amounts of from 0.05% to 3%, more preferably 0.1 % to 1% and most preferably 0 15% to 0.5% by weight of the composition

Antiredeposition and soil-suspension agents suitable herein include cellulose derivatives such as methylcellulose, carboxymethylcellulose and hydroxyethylcellulose, and homoorco-polymeric polycarboxylic acids or their salts in which the polycarboxylic acid comprises at least two carboxyl radicals separated from each other by not more than two carbon atoms. Polymers of this type are disclosed in GB-A-1,596,756. Preferred polymers include homopolymers of acrylic acid and copolymers of maleic anhydride with ethylene, methylvinyl ether, acrylic acid or methacrylic acid, the maleic anhydride constituting at least 20 mole percent of the copolymer. These materials are normally used at levels of from 0.5% to 10% by weight more preferably from 0.75% to 8%, most preferably from 1% to 6% by weight of the composition.

Other useful polymeric materials are the polyethylene glycols, particularly those of molecular weight 1000-10000, more particularly 2000 to 8000 and most preferably about 4000. These are used at levels of from 0.20% to 5% more preferably from 0.25% to 2.5% by weight.

All of the above polymers are valuable for improving whiteness maintenance, fabric ash deposition, and cleaning performance on clay, proteinaceous and oxidizable soils in the presence of transition metal impurities.

Fabric softening agents can also be incoporated into detergent compositions in accordance with the present invention. These agents may be inorganic or organic in type. Inorganic softening agents are exemplified by the smectite clays disclosed in GB-A-1,400,898. Organic fabric softening agents include the water-insoluble tertiary amines as disclosed in GB-A-1514276 and EP-B-0011340 and their combination with mono C₁₂C₁₄ quaternary ammonium salts are disclosed in EP-B-0026527 & 528 and dilong-chain amides as disclosed in EP-B-0242919. Other useful organic ingredients of fabric softening systems include high molecular weight polyethylene oxide materials as disclosed in EP-A-0299575 and 0313146.

Levels of smectite clay are normally in the range from 5% to 15%, more preferably from 8% to 12% by weight with the material being added as a dry mixed component to the remainder of the formulation. Organic fabric softening agents such as the water-insoluble tertiary amines or dilong-chain amide materials are incorporated at levels of from 0.5% to 5% by weight, normally from 1% to 3% by weight whilst the high molecular weight polyethylene oxide are materials and the water-soluble cationic materials are added at levels of from 0.1% to 2%, normally from 0.15% to 1.5% by weight These materials are normally added to the spray-dried portion of the composition, although in some instances it may be more convenient to add them as a dry mixed particulate, or spray them as a molten liquid on to other solid components of the composition.

Enzymes suitable for use herein include proteolytic, amylolytic and lipolytic enzymes. Examples of such enzymes and their levels of incorporation are given in U.S.P. 3,519,570 and U.S. P. 3,533,139. Photoactivated bleaching materials are discussed in EP-A-57088, highly preferred materials being zinc phthalocyanine tri- and tetra-sulphonates, such materials being present at from 0.0005% to 0.01 % by weight of the composition.

Anionic or nonionic optical brighteners (fluorescers) are also preferred ingredients of detergent compositions in accordance with the invention, being normally present at levels of from 0.01% to 1% by weight, more preferably at levels of from 0.02% to 0.5% by weight. A preferred fluorescer is the anionic material available from Ciba Geigy S.A. under the trade name Tinopal CBS and mixtures thereof with materials available under the trade names Tinopal EMS and Blankophor MBBN, the latter being sold by Farbenfabriken Bayer AG

In the detergent composition aspect of the invention the hereindefined peroxy acid precursors are normally employed at levels of from 1 % to 15% by weight, more preferably at from 1 % to 10%, and most frequently at from 2% to 5% by weight of the detergent composition.

The present invention also finds utility in the formation of aqueous bleaching liquors by means of the reaction of a source of alkaline hydrogen peroxide with organic peroxyacid precursors as hereinbefore defined either as is, or by means of the addition of the above-described detergent or bleaching additive compositions to an aqueous medium. It has been found that in addition to the source of hydrogen peroxide, normally present in amounts to provide from 50 to 350 ppm of available oxygen in the wash-liquor, the precursors should preferably be present in an amount to provide a level of from 1 to 50ppm of available oxygen in the wash-liquor. Preferably the amount should be such as to provide at least 2 ppm and more preferably at least 20 ppm, in order that the benefit of the peroxy acid can be realised.

The invention is illustrated in the following non-limiting examples in which all parts and percentages are by weight unless otherwise specified.

### EXAMPLE 1

### Perhydrolysis of Potassium Diacetyl Amino Ethane Sulphonate (KDAES) & Tetracetyl Ethylene Diamine (TAED) at 10°C.

The rates of perhydrolysis of KDAES (using the product from the preparation described hereinbefore) and TAED (supplied by Hoechst AG) were compared using material
a) predissolved
b) in solid form
   0.62g KDAES and 0.30g TAED were used in each leg of the test, with the KDAES being dissolved into 10ml distilled water and the TAED being dissolved in 10mi dimethyl formamide. The TAED was a particulate in which 95% by weight was less than 150 micrometers in size and no more than 30% was less than 32 micrometers. A jacketed 1 litre glass vessel fitted with a magnetic stirrer was used to carry out the perhydrolysis and the temperature of the solution was controlled at 10°C by means of a Thermomix (RTM) cooling unit which was used to circulate the cooled water through a jacket around the perhydrolysis vessel

### PROCEDURE

500ml distilled water, 1.5g sodium perborate tetrahydrate and 0.02g sodium ethylene diamine tetramethylene phosphonate were added to the vessel. A 1 Oml aliquot was withdrawn and a blank titration was carried out with no precursor present after which the precursor was added and successive 10ml aliquots withdrawn after 1,3,5,8 & 15 minutes, with a further aliquot being withdrawn for the TAED sample after 30 minutes. The extent of perhydrolysis was determined by withdrawing a 10 ml aliquot of the solution, adding it to 30 ml of chilled acidified (acetic acid) KI solution with 0.01 M sodium thiosulphate solution using an lotect indicator near the end-point. The value obtained for the blank was subtracted from the values obtained at the various time intervals to give the figure from which the percentage perhydrolysis was calculated.

### RESULTS

These are shown in Table 1. It can be seen that whereas the rates of perhydrolysis of the two precursors are broadly similar when each is predissolved, there is a marked difference between the rates of perhydrolysis when the precursors are added in solid form. If the KDAES is assumed to be 82% active (based on the level of the diacetyl derivative), then dissolution and perhydrolysis is substantially complete within 5 minutes whereas the TAED takes at least 30 minutes to reach a similar level of dissolution and perhydrolysis.

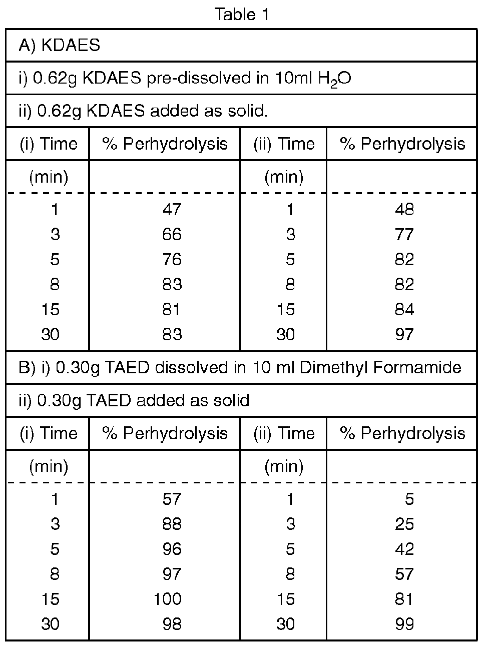

### EXAMPLE 2

### Perhydrolysis of N, N-diacetyl trimethyl ammonio propylamine bromide (DTAPB)

0.698g of the solid product from the three stage preparation described hereinbefore was subjected to the same perhydrolysis procedure as in Example 1.

The results were as follows

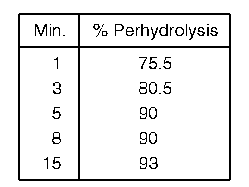

The purity of the DTAPB product was 97.6% and on this basis approx. 92% of the precursor perhydrolysed within 5 minutes.

### EXAMPLE 3

A comparison was made of the relative bleaching performance at 10°C of bleaching liquors containing respectively KDAES (using the material employed in Example 1), DTAPB (using the material employed in Example 2) and TAED in a model bleaching test, in which 6 mmoles of sodium perborate tetrahydrate and 3.5 mmoles of precursor were added to 500ml distilled water in a 1 000ml vessel containing cotton swatches bearing an EMPA wine-stain. Swatches were withdrawn after 3, 6 and 9 minutes for measurement of the stain removal by means of a light reflectance technique. At each time interval the stain removal provided by the bleaching liquors incorporating the precursors of the invention was superior to that provided by the corresponding liquor containing TAED.

### EXAMPLE 4

A comparison was made of the fabric colour damage potential of sodium perborate alone and mixtures of TAED- sodium perborate, and KDAES-sodium perborate using a model test to simulate stressed dissolution conditions

Nine 10cm x 7.5cm swatches were cut from a length of blue 100% lambswool cloth supplied by Borval Fabrics (Design No. W3970 Shade: Purple 48). The swatches were wetted, wrung out to remove surplus moisture, smoothed and then immersed so as to lie flat in individual dishes each containing 100mls of a 3% (by wt.) sodium perborate solution at 20°C. The solution had a pH of 9.3.

0.01g of powdered TAED was sprinkled on to each of three of the immersed swatches and 0.01 of powdered KDAES was sprinkled on to each of another three of the immersed swatches, which were then left for 24 hrs.

At the end of this period the pH of the solutions. was measured and the swatches were visually examined. The pH of the perborate solution remained at approximately 9.3 and the swatches had changed colour uniformly from deep blue to a sage green. The TAED containing solutions had an average pH of 8.6 and the swatches had changed colour to a greenish-blue which was very uneven, showing evidence of 'pinpoint' colour change where the TAED had either not dissolved or dissolved more slowly. The KDAES solutions had an average pH of 8.2 and the swatches showed little fading from the original blue colour and little unevenness in hue variation, indicating substantially complete and rapid perhydrolysis of the bleach precursor.

This demonstrates that aqueous bleaching liquors incorporating inorganic perhydrates and peroxyacid bleach precursors in accordance with the invention cause less colour damage to fabrics immersed therein than bleaching liquors containing prior art precursors or inorganic perhydrates alone.

Various detergent composition embodiments of the invention are shown below in which the abbreviated component identifications have the following meanings:

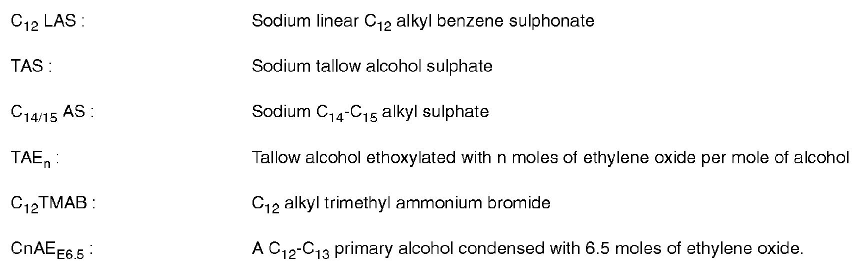

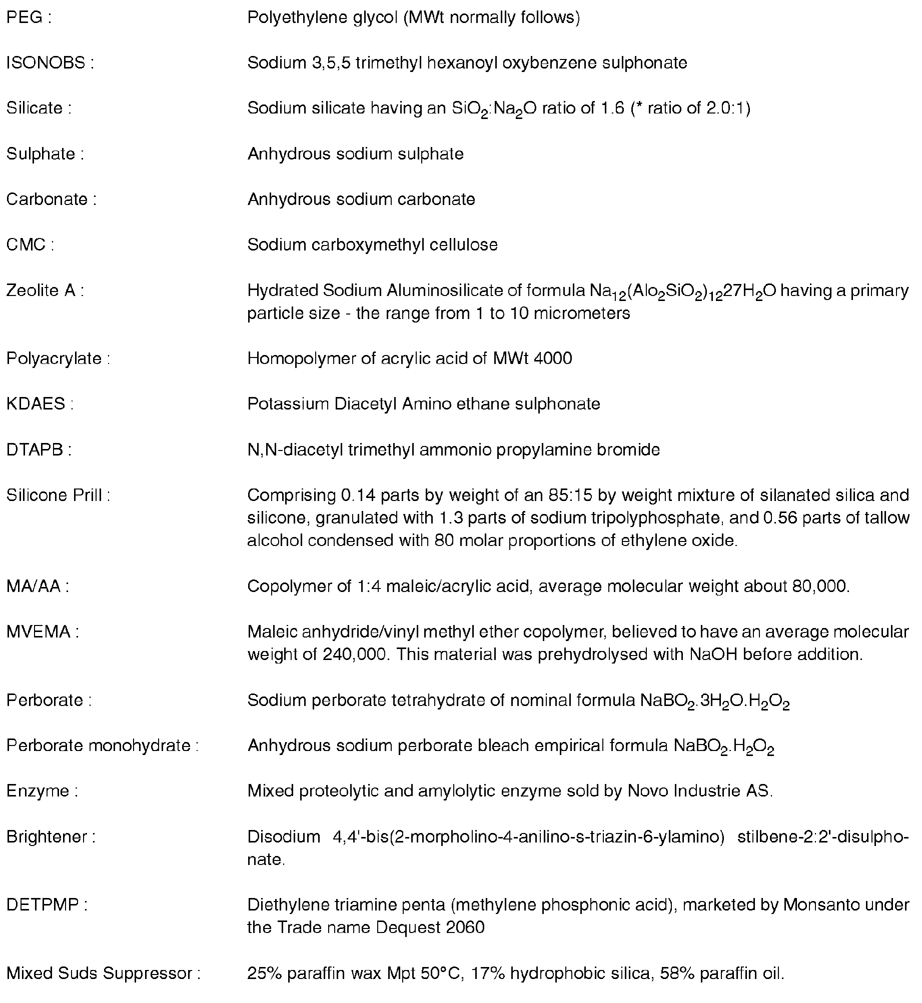

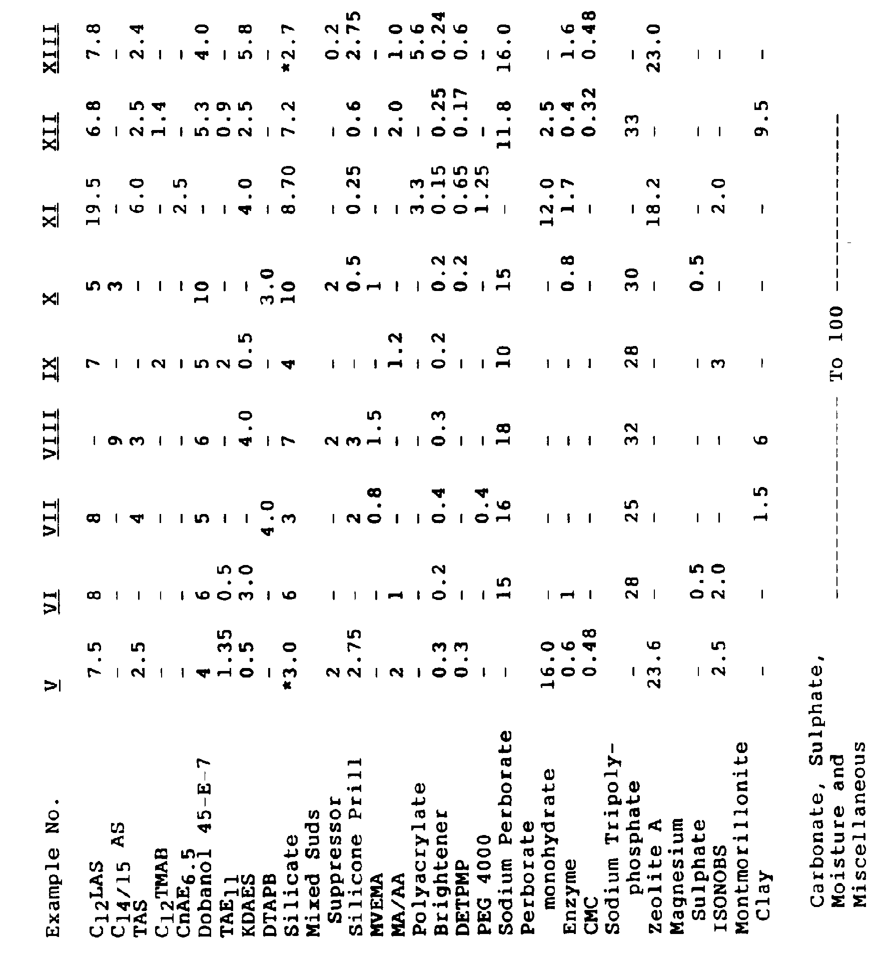

The phosphate-containing products are made by preparing a spray-dried base powder containing the anionic and any cationic surfactants, STP, inorganic salts, brightener, copolymer and DETPMP and then the remaining ingredients are incorporated by spray on to the base powder (nonionic), dry mixing (perborate) or via prill addition (peroxy acid precursor, silicone and enzyme).

The zeolite-containing products V, XI & XIII are made in a similar manner except that the sodium silicate does not form part of the spray-dried powder but is added subsequently as a powder.

In composition XIII, 6.0 parts of the LAS, 8.5 parts of sodium carbonate and 6.0 parts of zeolite builder are formed into an agglomerate and added as a dry mixed component to the spray-dried portion and the remaining ingredients.

## Claims

1. A water-soluble peroxyacid bleach precursor having the general formula: 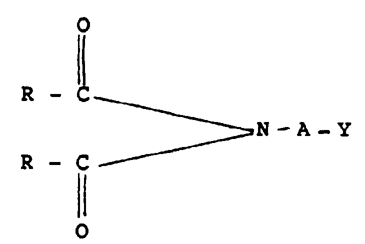 wherein each
R is CH₃- or C₂H₅-
A is C₂-C₆ alkylene or a phenylene group
Y is
- OSO₃M+
- SO₃M+
- N⁺R₁R₂R₃X- 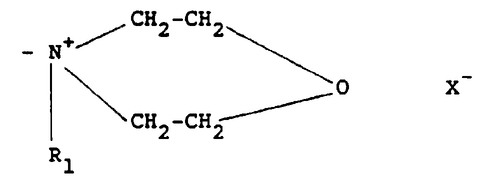 wherein M⁺ is hydrogen alkali metal or alkaline earth metal, R_{1'} R₂ & R₃ are independently C₁-C₄ alkyl and X- is halide or methosulphate.

2. A peroxyacid bleach precursor according to claim 1 wherein R is CH₃- and A is a C₂-C₃ alkylene group.

3. A peroxyacid bleach precursor according to claim 2 wherein Y is -SO₃M+ or -N+R₁R₂R₃X-

4. A peroxyacid bleach precursor comprising the sodium or potassium salt of di-N-acetyl taurine.

5. A peroxyacid bleach precursor comprising N,N-diacetyl trimethylammoniopropylamine bromide or chloride.

6. A method of preparing a N,N-diacetyl C₁-C₄ ammonio C₂-C₆ alkyl amine bromide or chloride comprising the steps of
i) reacting a di C₁-C₄ alkylamino C₂-C₆ alkylene amine with acetic anhydride in an amount of at least two moles acetic anhydride per mole of amine, under conditions of reflux;
ii) removing the acetic acid by-product from the reaction mixture by distillation
iii) adding a C₁-C₄ alkyl chloride or bromide to the reaction mixture from step ii); and
iv) recovering the solid N,N-diacetyl C₁-C₄ ammonio C₂-C₆ alkylamine bromide or chloride product.

7. A process according to claim 6 wherein the C₁-C₄ alkyl moiety in the starting amine and in the alkyl halide is a methyl group.

8. A process according to claim 7 wherein the C₂-C₆ alkylene group is a n-propylene group.

9. A process for preparing a di-N-acetyl amino C₂ C₆ alkyl sulphonate comprising the steps of
i) reacting an amino C₂-C₆ alkanesulphonic acid with at least two moles of acetic anhydride and at least an equimolar amount of triethylamine, said reaction mixture being heated to remove the acetic acid by-product;
ii) adding an alkali metal acetate to the reaction mixture of step i) in a molar amount at least equal to that of the acetic acid by-product resulting from step i).
iii) Cooling the mixture of step ii) to precipitate the di-N-acetyl amino C₂-C₆ alkanesulphonate and
iv) recovering the di-N-acetyl amino C₂-C₆ alkanesulphonate product.

10. A process according to claim 9 wherein, in step ii) the alkali metal acetate is added as a saturated solution in acetic acid.

11. A process according to either one of claims 9 & 10 wherein the C₂-C₆ alkyl moiety is an ethyl group.

12. An aqueous bleaching liquor for the bleaching of oxidisable stains on surfaces exposed thereto at temperatures of 60°C and below comprising a mixture of a source of alkaline hydrogen peroxide in an amount to provide from 50 to 350 ppm of available oxygen and a water-soluble peroxyacid precursor as defined in any one of claims 1-5 in an amount to produce from 1 to 50 ppm (theoretical) of the peroxyacid.

13. A solid composition adapted to provide bleaching of oxidisable stains on surfaces when mixed with an alkaline aqueous source of hydrogen peroxide at temperatures of 60°C and below comprising a peroxyacid bleach precursor according to anyone of claims 1-5 in combination with one or more components selected from agglomeration and/or coating agents, surfactants, builder salts, chelating agents, soil-suspending agents, suds-modifying agents, polymeric soil-release agents, fabric-softening agents, fluorescers and perfumes.

14. A solid composition according to claim 13 wherein the peroxyacid bleach precursor is in admixture with an ethoxylated nonionic surfactant, preferably in the form of an extrudate or agglomerate.

15. A solid detergent composition according to either one of claims 13 & 14 incorporating a source of hydrogen peroxide in the form of a particulate inorganic compound or a hydrogen peroxide inclusion compound

16. A solid detergent composition according to claim 15 comprising:
a) a peroxyacid bleach precursor in the form of an extrudate or an agglomerate
b) a source of hydrogen peroxide in the form of a particulate inorganic perhydrate salt
c) at least one particulate detergent component selected from surfactants, builder salts, chelating agents, soil-suspending agents, suds-modifying agents, polymeric soil-release agents, fabric-softening agents, fluorescers, perfumes and mixtures thereof.

17. A method of bleaching oxidisable stains on fabric surfaces in an aqueous bath of a temperature of 20°C and below providing a mixture of a source of alkaline hydrogen peroxide and a water-soluble peroxyacid bleach precursor as defined in any one of claims 1-5 in an amount to produce from 1 to 50ppm (theoretical) of the peroxyacid.

18. A method of bleaching oxidisable stains according to claim 16 wherein the mixture comprises a composition according to either one of claims 15 and 16.

## Patentansprüche

1. Wasserlöslicher Peroxysäure-Bleichmittelvorläufer der allgemeinen Formel: 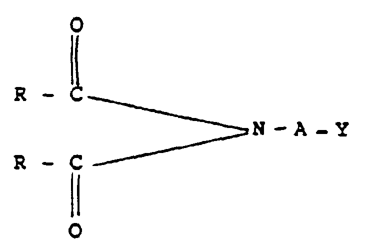 worin bedeuten:
R jeweils CH₃- oder C₂H₅-
A eine C₂-C₆-Alkylen- oder eine Phenylengruppe
Y - OSO₃⁻M+
- SO₃⁻M⁺
- N⁺R₁ R₂R₃X⁻ 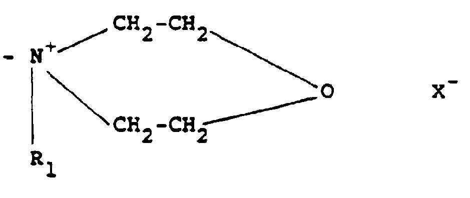 worin M⁺ Wasserstoff, ein Alkalimetall oder Erdalkalimetall ist, R₁, R₂ und R₃ unabhängig voneinander C₁-C₄-Alkyl sind und X- ein Halogenid oder Methosulfat ist.

2. Peroxysäure-Bleichmittelvorläufer nach Anspruch 1, wobei R CH₃- ist und A eine C₂-C₃-Alkylengruppe ist.

3. Peroxysäure-Bleichmittelvorläufer nach Anspruch 2, wobei Y -SO₃⁻M⁺ oder -N+R₁R₂R₃X⁻ ist.

4. Peroxysäure-Bleichmittelvorläufer, umfassend das Natrium- oder Kaliumsalz von Di-N-acetyltaurin.

5. Peroxysäure-Bleichmittelvorläufer, umfassend N,N-Diacetyltrimethylammoniumpropylaminbromid oder -chlorid.

6. Verfahren zur Herstellung eines N,N-Diacetyl-C₁-C₄-ammonium-C₂-C₆-alkylaminbromids oder -chlorids, umfassend die Schritte
(i) Umsetzen eines Di-C₁-C₄-alkylamino-C₂-C₆-alkylenamins mit Essigsäureanhydrid in einer Menge von mindestens 2 Molen Essigsäureanhydrid pro Mol Amin unter Rückflußbedingungen;
(ii) Entfernen des Essigsäure-Nebenprodukts aus der Reaktionsmischung duch Destillation;
(iii) Zugeben eines C₁-C₄-Alkylchlorids oder -bromids zu der Reaktionsmischung aus Schritt (ii); und
(iv) Gewinnen des festen N,N-Diacetyl-C₁-C₄-ammonium-C₂-C₆-alkylaminbromid- oder -chlorid-Produkts.

7. Verfahren nach Anspruch 6, wobei die C₁-C₄-Alkylgruppe in dem Ausgangsamin und in dem Alkylhalogenid eine Methylgruppe ist.

8. Verfahren nach Anspruch 7, wobei die C₂-C₆-Alkylengruppe eine n-Propylengruppe ist.

9. Verfahren zur Herstellung eines Di-N-Acetylamino-C₂-C₆-alkylsulfonats, umfassend die Schritte
(i) Umsetzen einerAmino-C₂-C₆-alkansulfonsäure mit mindestens 2 Molen Essigsäureanhydrid und mindestens einer äquimolaren Menge Triethylamin, wobei die Reaktionsmischung zur Entfernung des Essigsäure-Nebenprodukts erwärmt wird;
(ii) Zugeben eines Alkalimetallacetats zu der Reaktionsmischung aus Schritt (i) in einer molaren Menge, die zumindest gleich derjenigen des Essigsäure-Nebenprodukts, das aus Schritt (i) resultiert, ist;
(iii) Kühlen der Mischung aus Schritt (ii) zur Ausfällung des Di-N-Acetylamino-C2-C6-alkansulfonats; und
(iv) Gewinnen des Di-N-Acetylamino-C₂-C₆-alkansulfonat-Produkts.

10. Verfahren nach Anspruch 9, wobei in Schritt (ii) das Alkalimetallacetat als eine gesättigte Lösung in Essigsäure zugegeben wird.

11. Verfahren nach einem der Ansprüche 9 und 10, wobei die C₂-Cᵣ,-Alkylgruppe eine Ethylgruppe ist.

12. Wäßrige Bleichflotte für das Bleichen von oxidierbaren Flecken auf dieser ausgesetzten Oberflächen bei Temperaturen von 60°C und darunter, umfassend eine Mischung aus einer alkalischen Wasserstoffperoxid-Quelle in einer Menge , um 50 bis 350 ppm verfügbaren Sauerstoff vorzusehen, und einem wasserlöslichen Peroxysäure-Vorläufer nach mindestens einem der Ansprüche 1-5 in einer Menge, um 1 bis 50 ppm (theoretisch) der Peroxysäure zu erzeugen.

13. Feststoffzusammensetzung, welche so ausgelegt ist, daß sie eine Bleichung oxidierbarer Flecken auf Oberflächen bei Vermischung mit einer alkalischen wäßrigen Wasserstoffperoxid-Quelle bei Temperaturen von 60°C und darunter ergibt, umfassend einen Peroxysäure-Bleichmittelvorläufer nach mindestens einem der Ansprüche 1-5 in Kombination mit einer oder mehreren Komponenten, gewählt aus Agglomerations- und/oder Beschichtungsmitteln, Tensiden, Buildersalzen, Komplexbildnern, Schmutzsuspendiermitteln. Schaummodifiziermitteln, polymeren Schmutzabweisungsmitteln. Gewebeweichmachern, Fluoreszenzmitteln und Duftstoffen.

14. Feststoffzusammensetzung nach Anspruch 13, wobei der Peroxysäure-Bleichmittelvorläufer in Mischung mit einem ethoxylierten nichtionischen Tensid vorliegt, vorzugsweise in Form eines Extrudats oder Agglomerats.

15. Feste waschmittelzusammensetzung nach einem der Ansprüche 13 und 14, beinhaltend eine Wasserstoffperoxid-Quelle in Form einer teilchenförmigen anorganischen Verbindung oder einer wasserstoffperoxid-Einschlußverbindung.

16. Feste Waschmittelzusammensetzung nach Anspruch 15, umfassend:
(a) einen Peroxysäure-Bleichmittelvorläufer in Form eines Extrudats oder eines Agglomerats,
(b) eine Wasserstoffperoxid-Quelle in Form eines teilchenförmigen anorganischen Perhydratsalzes,
(c) mindestens eine teiichenförmigeWaschmitteikomponente, gewählt aus Tensiden, Buildersalzen, Komplexbildnern, Schmutzsuspendiermitteln, Schaummodifiziermitteln, polymeren Schmutzabweisungsmitteln, Gewebeweichmachern, Fluoreszenzmitteln, Duftstoffen und Mischungen hiervon.

17. Verfahren zum Bleichen oxidierbarer Flecken auf Gewebeoberflächen in einem wäßrigen Bad bei einer Temperatur von 20°C und darunter, bei dem eine Mischung aus einer alkalischen Wasserstoffperoxid-Quelle und einem wasserlöslichen Peroxysäure-Bleichmittelvorläufer nach mindestens einem der Ansprüche 1-5 in einer Menge zur Erzeugung von 1 bis 50 ppm (theoretisch) der Peroxysäure vorgesehen wird.

18. Verfahren zum Bleichen oxidierbarer Flecken nach Anspruch 17, wobei die Mischung eine Zusammensetzung nach einem der Ansprüche 15 und 16 enthält.

## Revendications

1. Précurseur de blanchiment de peroxyacide soluble dans l'eau ayant pour formule générale : 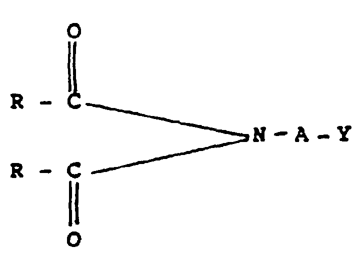 dans laquelle chaque groupe est
R CH₃- ou C₂H₅-
A est un groupe alkylène en C₂-C₆ ou un groupe phénylène
Y est
-OS0₃-M⁺
-S0₃-M⁺
N⁺R₁R₂R₃X⁻ 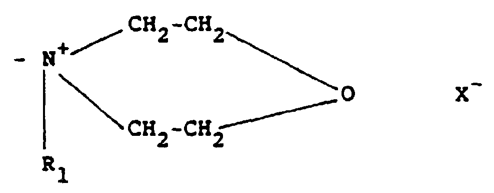 où M⁺ est de l'hydrogène, un métal alcalin ou un métal alcalino-terreux, R₁, R₂ et R₃ sont indépendamment un groupe alkyle en C₁-C₄ et X⁻ est un halogénure ou un méthosulfate.

2. Précurseur d'agent de blanchiment de peroxyacide selon la revendication 1, dans lequel R est CH₃- et A est un groupe alkylène en C₂-C₃.

3. Précurseur d'agent de blanchiment de peroxyacide selon la revendication 2, dans lequel Y est -SO₃⁻M⁺ ou -N⁺R₁R₂R₃X⁻.

4. Précurseur d'agent de blanchiment de peroxyacide comprenant le sel de sodium ou de potassium de la di-N-acétyl taurine.

5. Précurseur d'agent de blanchiment de peroxyacide comprenant le bromure ou le chlorure de N,N-diacétyl trimé- thylammoniopropylamine.

6. Procédé de préparation d'un bromure ou d'un chlorure de N,N-diacétyl C₁-C₄ ammonio C₂-C₆ alkyl amine comprenant les stades suivants :
i) on fait réagir une di C₁-C₄ alkylamino C₂-C₆ alkylène amine avec de l'anhydride acétique en quantité d'au moins deux moles d'anhydride acétique par mole d'amine dans des conditions de reflux,
ii) on élimine le sous-produit d'acide acétique du mélange réactionnel par distillation,
iii) on ajoute du chlorure ou du bromure d'alkyle en C₁-C₄ au mélange réactionnel du stade ii), et
iv) on récupère le bromure ou le chlorure solide de N,N-diacétyl C₁-C₄ ammonio C₂-C₆ alkylamine produit.

7. Procédé selon la revendication 6, dans lequel le radical alkyle en C₁-C₄ dans l'amine de départ et dans l'halogénure d'alkyle est le groupe méthyle.

8. Procédé selon la revendication 7, dans lequel le groupe alkylène en C₂-C₆ est le groupe n-propylène.

9. Procédé de préparation de di-N-acétyl amino C₂-C_{S} alkyl sulfonate comprenant les stades suivants :
i) on fait réagir un acide amino alcanesulfonique en C₂-C_{S} avec au moins deux moles d'anhydride acétique et au moins une quantité équimolaire de triéthylamine, ledit mélange réactionnel étant chauffé pour éliminer le sous-produit d'acide acétique,
ii) on ajoute un acétate de métal alcalin au mélange réactionnel du stade i) en quantité molaire au moins égale à celle du sous-produit d'acide acétique résultant du stade i);
iii) on refroidit le mélange du stade ii) pour précipiter le di-N-acétyl amino C₂-C₆ alcanesulfonate, et
iv) on récupère le di-N-acétyl amino C₂-C_{S} alcane sulfonate produit.

10. Procédé selon la revendication 9, dans lequel, au stade ii), l'acétate de métal alcalin est ajouté sous la forme d'une solution saturée dans de l'acide acétique.

11. Procédé selon l'une ou l'autre des revendications 9 et 10, dans lequel le radical alkyle en C₂-C_{G} est un groupe éthyle.

12. Liqueur de blanchiment aqueuse pour le blanchiment de taches oxydables sur des surfaces exposées à cette liqueur à des températures de 60°C et en dessous, comprenant un mélange d'une source de peroxyde d'hydrogène alcalin en quantité pour fournir 50 à 350 ppm d'oxygène disponible et d'un précurseur de peroxyacide soluble dans l'eau tel que défini dans l'une quelconque des revendications 1 à 5 en quantité susceptible de produire 1 à 50 ppm (en théorie) du peroxyacide.

13. Composition solide susceptible d'assurer le blanchiment de taches oxydables sur des surfaces lorsqu'elle est mélangée à une source alcaline aqueuse de peroxyde d'hydrogène à des températures de 60°C et en dessous, comprenant un précurseur d'agent de blanchiment de peroxyacide selon l'une quelconque des revendications 1 à 5 en combinaison avec un ou plusieurs composants sélectionnés parmi des agents d'agglomération et/ou de revêtement, des agents tensioactifs, des sels d'adjuvants, des agents chélatants, des agents de suspension de crasses, des agents modificateurs de mousses, des agents de libération de crasses polymères, des agents adoucisseurs de tissus, des agents fluorescents et des parfums.

14. Composition solide selon la revendication 13, dans laquelle le précurseur d'agent de blanchiment de peroxyacide est présent en mélange avec un agent tensioactif non ionique éthoxylé, de préférence sous la forme d'un extrudat ou d'un agglomérat.

15. Composition détergente solide selon l'une ou l'autre des revendications 13 et 14, contenant une source de peroxyde d'hydrogène sous la forme d'un composé particulaire minéral ou un composé d'inclusion de peroxyde d'hydrogène.

16. Composition détergente solide selon la revendication 15, comprenant :
a) un précurseur d'agent de blanchiment de peroxyacide sous la forme d'un extrudat ou d'un agglomérat,
b) une source de peroxyde d'hydrogène sous la forme d'un sel perhydraté minéral particulaire,
c) au moins un composant détergent particulaire sélectionné parmi les agents tensioactifs, les sels d'adjuvants, les agents chélatants, les agents de suspension de crasses, les agents de modification de mousses, les agents de libération de crasses polymères, les agents adoucisseurs de tissus, les agents fluorescents, les parfums et leurs mélanges.

17. Procédé de blanchiment de taches oxydables sur des surfaces de tissus dans un bain aqueux, à une température de 20°C et en dessous, formant un mélange d'une source de peroxyde d'hydrogène alcalin et d'un précurseur d'agent de blanchiment de peroxyacide soluble dans l'eau tel que défini dans l'une quelconque des revendications 1 à 5 en quantité permettant de produire 1 à 50 ppm (en théorie) du peroxyacide.

18. Procédé de blanchiment de taches oxydables selon la revendication 16, dans lequel le mélange comprend une composition selon l'une ou l'autre des revendications 15 et 16.
